# EUROPEAN PATENT APPLICATION

(11) **EP 4 273 155 A1**
(43) Date of publication of application: **08.11.2023**
(21) Application number: 21914021.7
(22) Date of filing: 21.12.2021
(51) Int. Cl.: C07K 14/08, A61K 35/76, A61K 39/12, A61P 31/14

(54) **NOROVIRUS VIRUS-LIKE PARTICLE, IMMUNE COMPOSITION OR KIT, AND USE THEREOF**

(30) Priority: 30.12.2020 CN 202011606203; 30.12.2020 CN 202011606268
(71) Applicant: Grand Theravac Life Science (Nanjing) Co., Ltd., Nanjing, Jiangsu 210032 (CN)
(72) Inventor: LI, Jianqiang, Nanjing, Jiangsu 210032 (CN); GE, Jun, Nanjing, Jiangsu 210032 (CN); WANG, Xiaodong, Nanjing, Jiangsu 210032 (CN); TAN, Changyao, Nanjing, Jiangsu 210032 (CN); REN, Sulin, Nanjing, Jiangsu 210032 (CN); ZHOU, Tong, Nanjing, Jiangsu 210032 (CN); GU, Yue, Nanjing, Jiangsu 210032 (CN); CHEN, Yue, Nanjing, Jiangsu 210032 (CN); HUANG, Hongying, Nanjing, Jiangsu 210032 (CN); CHEN, Xiaoxiao, Nanjing, Jiangsu 210032 (CN)
(74) Representative: Uexküll & Stolberg
(86) International application number: PCT/CN2021/139930
(87) International publication number: WO 2022/143282

(57) **Abstract**

Provided is a G11.4 norovirus virus-like particle or active fragment thereof, and use thereof, the virus-like particle comprising or being composed of an amino acid sequence shown as SEQ ID NO: 4. Further, provided is a composition or a kit comprising the G11.4 norovirus virus-like particle, and use thereof. The objective of covering multiple genotypes is achieved by using a single antigen of the invention, the process difficulty of the preparation for a pharmaceutical composition or a vaccine can be reduced, and the production cost is decreased. Also, provided is a norovirus immune composition or a kit comprising GII.2, GII.4, GII.6, and GII.17 norovirus virus-like particles or active fragments thereof, and use thereof. By the composition or the kit comprising the four types of antigens provied by the invention, each of antigens can generate a cross-immune effect on other genotypes, not only multiple prevailing genotypes of noroviruses can be covered, but also a synergy of immune effects on the same genotypes to which the four types of antigens target.

## Description

### Technical Field

The invention belongs to the field of biopharmaceutics, and relates to a norovirus virus-like particle, and a composition or a kit for inducing an immune response to a norovirus. The invention also provides use of the norovirus virus-like particle, the composition or the kit.

### Background Art

In 1972, norovirus was first discovered by the American scholar Kapikian in the feces of diarrhea patients in Norwalk Town and named as Norwalk Virus (NoV), and then as norovirus by the Eighth International Committee on Nomenclature of Viruses. It is one of the main pathogens causing human infectious gastroenteritis outbreaks and acute diarrhea in infants and young children.

Norovirus is a single-stranded positive-strand RNA virus belonging to Caliciviridae, which is approximately 26-35 nm in diameter, non-enveloped, rough-surfaced, spherical, with an icosahedral symmetry, and isolated from the feces of patients with acute gastroenteritis. Norovirus genome is approximately 7.7 kb in full length and contains three open reading frames (ORFs). ORF1 encodes nonstructural proteins, including RNA polymerase (RNA-dependent RNA polymerase, rdRp). ORF2 and ORF3 encode major (VP1) and minor (VP2) capsid proteins, respectively. According to the phylogenetics of gene sequences, norovirus can be divided into 6 genogroups (GI-GVI), and each genegroup can be further divided into multiple genotypes. Human infection is mainly caused by GI and GII genegroups, wherein GII group is the dominant one. Statistically, more than 75% of human NoV acute gastroenteritis was caused by the infections of GII group.

In different periods and regions, the detection rate and prevalence of various genotypes have major differences and changes. In the recent 20 years, GII.4 genotype has been in a dominant position in the outbreaks and sporadic cases worldwide, but the diversity of genotypes and rapid variation of epidemic strains are important characteristics of norovirus. In different periods and regions, the detection rate and prevalence of various genotypes have great differences and changes. In particular, GII. 2, GII. 3, GII. 6, GII. 17, etc. have a higher detection rate among the genotypes other than GII. 4. In recent years, with the change of epidemic trend, they also triggered small-scale epidemics in local areas.

Norovirus, which is characterized by the diversity and rapid variation of genotypes, has no an ideal cell culture system and animal model, and thus the development of anti-norovirus drugs was limited to some extent. There is still lack of effective drugs, vaccines and preventive measures. Therefore, the development of effective anti-viral drugs and prophylactic vaccines is currently a focus problem to be solved urgently.

Available research data show that there is no antibody cross-reactivity and cross-blocking activity between GI and GII genogroups of norovirus (Han Zibo, Zhang Xuefeng, Liu Zhaoming, et al., Chinese Journal of Biologicals, 2019). Thus, current research on vaccines against multiple genotypes of noroviruses has been focusing on a variety of artificially synthesized polypeptides.

Based on this, there is currently a need for vaccines against multiple genotypes of noroviruses.

### Contents of the Invention

It is therefore an object of the invention to provide a norovirus virus-like particle, an immune composition or a kit for multiple genotypes of noroviruses and use thereof in view of the deficiencies in the prior art.

The inventors have conducted extensive studies on the cross immunities between various genotypes of norovirus antigens, and surprisingly found that GII. 4 norovirus virus-like particles having a stronger cross-immune effect on GI. 1, simultaneously have cross-immune effects on GII. 2, GII. 7 and GII. 12, etc. Based on this finding, the inventors provide a G11.4 norovirus virus-like particle (VLP), an immune composition or a kit against a norovirus, and use thereof.

The inventors have also unexpectedly obtained a composition or a kit for inducing an immune response to a norovirus, comprising GII. 2, GII. 4, GII. 6 and GII. 17 norovirus virus-like particles. The pharmaceutical composition or the kit can cover both GI. 1 and GII. 7, and also has a cross-immune effect on GII. 12. By using the composition or the kit of the invention, it is possible to achieve a goal of covering 7 genotypes with 4 antigens, and there is also a superposition of immune effects on the same genotypes to which the four antigens target.

The object of the invention is achieved by the following technical solutions.

In an aspect, the invention provides a G11.4 norovirus virus-like particle, or active fragment thereof for inducing an immune response to multiple genotypes of noroviruses in an animal, wherein the G11.4 norovirus virus-like particle or active fragment thereof comprises or is composed of one amino acid sequence selected from a group consisting of:
(1) an amino acid sequence shown as SEQ ID NO: 4;
(2) an amino acid sequence having an identity of at least 90%, 91%, 92%, 93%, 94%, 95%, 96%, 97%, 98%, 99% or more to the amino acid sequence shown as SEQ ID NO: 4;
(3) an amino acid sequence having an amino acid substitution, deletion or insertion at one or more sites in the amino acid sequence shown as SEQ ID NO: 4; and
(4) a segment of consecutive amino acids from position X to position 480 in the amino acid sequence shown as SEQ ID NO: 4, wherein X is an integer between 2 and 39; preferably, X is 27, 37 or 39.

Preferably, the animal is a mammal, more preferably a human; and/or the multiple genotypes of noroviruses include two or more selected from a group consisting of GI. 1, GII. 2, GII. 4, GII. 7 and GII. 12 noroviruses.

In another aspect, the invention provides a composition for inducing an immune response to a norovirus in an animal, comprising the G11.4 norovirus virus-like particle or active fragment thereof as described herein.

Preferably, the animal is a mammal, more preferably a human.

Preferably, the norovirus is one or more selected from a group consisting of GI. 1, GII. 2, GII. 4, GII. 7 and GII. 12 noroviruses.

In yet another aspect, the invention provides a composition for inducing an immune response to a norovirus, comprising GII. 2, GII. 4, GII. 6 and GII. 17 noroviruses virus-like particles or active fragments thereof.

In a preferred embodiment of the invention, the GII. 2 norovirus virus-like particle or active fragment thereof comprises or is composed of one amino acid sequence selected from a group consisting of:
(1) an amino acid sequence shown as SEQ ID NO: 2;
(2) an amino acid sequence having an identity of at least 90%, 91%, 92%, 93%, 94%, 95%, 96%, 97%, 98%, 99% or more to the amino acid sequence shown as SEQ ID NO: 2; and
(3) an amino acid sequence having an amino acid substitution, deletion or insertion at one or more sites in the amino acid sequence shown as SEQ ID NO: 2.

Preferably, the GII.4 norovirus virus-like particle or active fragment thereof comprises or is composed of one amino acid sequence selected from a group consisting of:
(1) an amino acid sequence shown as SEQ ID NO: 4;
(2) an amino acid sequence having an identity of at least 90%, 91%, 92%, 93%, 94%, 95%, 96%, 97%, 98%, 99% or more to the amino acid sequence shown as SEQ ID NO: 4;
(3) an amino acid sequence having an amino acid substitution, deletion or insertion at one or more sites in the amino acid sequence shown as SEQ ID NO: 4; and
(4) a segment of consecutive amino acids from position X to position 480 in the amino acid sequence shown as SEQ ID NO: 4, wherein X is an integer between 2 and 39; preferably, X is 27, 37 or 39.

Preferably, the GII. 6 norovirus virus-like particle or active fragment thereof comprises or is composed of one amino acid sequence selected from a group consisting of:
(1) an amino acid sequence shown as SEQ ID NO: 5;
(2) an amino acid sequence having an identity of at least 90%, 91%, 92%, 93%, 94%, 95%, 96%, 97%, 98%, 99% or more to the amino acid sequence shown as SEQ ID NO: 5;
(3) an amino acid sequence having an amino acid substitution, deletion or insertion at one or more sites in the amino acid sequence shown as SEQ ID NO: 5; and
(4) a segment of consecutive amino acids from position X to position 547 in the amino acid sequence shown as SEQ ID NO: 5, wherein X is an integer between 2 and 27; preferably, X is 27.

Preferably, the GII. 17 norovirus virus-like particle or active fragment thereof comprises or is composed of one amino acid sequence selected from a group consisting of:
(1) an amino acid sequence shown as SEQ ID NO: 8;
(2) an amino acid sequence having an identity of at least 90%, 91%, 92%, 93%, 94%, 95%, 96%, 97%, 98%, 99% or more to the amino acid sequence shown as SEQ ID NO: 8; and
(3) an amino acid sequence having an amino acid substitution, deletion or insertion at one or more sites of the amino acid sequence shown as SEQ ID NO: 8.

More preferably, the GII. 2 norovirus virus-like particle or active fragment thereof comprises or is composed of the amino acid sequence shown as SEQ ID NO: 2; the GII.4 norovirus virus-like particle or active fragment thereof comprises or is composed of the amino acid sequence shown as SEQ ID NO: 4; the GII. 6 norovirus virus-like particle or active fragment thereof comprises or is composed of the amino acid sequence shown as SEQ ID NO: 5; and the GII. 17 norovirus virus-like particle or active fragment thereof comprises or is composed of the amino acid sequence shown as SEQ ID NO: 8.

In the composition according to the invention, the norovirus is one or more selected from a group consisting of GI. 1, GII. 2, GII. 4, GII. 6, GII. 7, GII. 12 and GII. 17 noroviruses.

Preferably, the composition according to the invention is a pharmaceutical composition, such as a vaccine, for the prevention and/or treatment of a norovirus infection.

Preferably, the pharmaceutical composition further comprises one or more adjuvant selected from a group consisting of an aluminum adjuvant, a TRL adjuvant, and a saponin adjuvant. More preferably, the aluminum adjuvant is one or more selected from a group consisting of aluminum hydroxide, aluminum phosphate and aluminum sulphate; further preferably, the aluminum adjuvant is aluminum hydroxide. More preferably, the TRL adjuvant is TRL9 adjuvant; further preferably, the TRL9 adjuvant is a CPG adjuvant; more fruther preferably, the nucleotide sequence of the CPG adjuvant is one selected from a group consisting of SEQ ID NO: 9, SEQ ID NO: 10 and SEQ ID NO: 11. More preferably, the saponin adjuvant is QS21 or Iscom adjuvant.

In the composition according to the invention, the norovirus infection may be gastroenteritis, preferably viral acute gastroenteritis.

In still another aspect, the invention also provides an immune kit comprising the norovirus virus-like particle or active fragment thereof according to the invention, or the composition according to the invention. Preferably, the kit is a kit for detecting a norovirus infection.

In still another aspect, the invention also provides use of the norovirus virus-like particle or active fragment thereof according to the invention, the composition according to the invention, or the kit according to the invention, in the preparation of a product as follows:
(1) a medicament for the prophylaxis and/or treatment of a norovirus infection;
(2) a kit for diagnosing a norovirus infection; or
(3) an immunogen for the development of a norovirus antibody.

In the use according to the invention, the norovirus infection may be gastroenteritis, preferably viral acute gastroenteritis.

In still another aspect, the invention provides a method for inducing an immune response to multiple genotypes of noroviruses in an animal, comprising administering the GII. 4 virus-like particle or active fragment thereof according to the invention to the animal in need. Preferably, the multiple genotypes of noroviruses include two or more selected from a group consisting of GI. 1, GII. 2, GII. 4, GII. 7 and GII. 12 noroviruses.

In another aspect, the invention also provides a method for inducing an immune response to noroviruses in an animal, comprising administering the composition of the invention to the animal in need.

The inventors have found that inclusion of the virus-like particles of GII. 4 norovirus of the amino acid sequence according to the invention produced cross-immune responses on the four genotypes of noroviruses, GI. 1, GII. 2, GII. 7 and GII. 12, which not only achieves cross-immunity between GII and GI genomes, but also has significant cross-immune effects on other genotypes within the genogroup, attaining a goal of preventing the infections by multiple genotypes of noroviruses using one antigen. The use of a single antigen of the invention to cover multiple genotypes can reduce the process difficulty in the preparation of a pharmaceutical composition or a vaccine, decrease the production cost, and has a broad market prospect.

The inventors of the invention have also found that a composition comprising the virus-like particles of GII. 2, GII. 4, GII. 6 and GII. 17 noroviruses can cover GI. 1, and also has cross-immune effects on GII. 7 and GII. 12. It not only achieves a goal of covering 7 genotypes by 4 antigens, but also has a superposition of immune effects for the same genotypes to which the four antigens target, i.e., the composition of the invention has synergy of immune effects on the same genotypes to which the four antigens target. For example, both GII. 4 and GII. 6 can cover GII. 7 at the same time, resulting in a significant increase of antibody titer compared to GII. 4 alone. Thus, the use of the composition of the invention can simultaneously cover a variety of norovirus genotypes, reduce the process difficulty in the preparation of a medicine or a vaccine, decrease the production cost, and has a broad market prospect.

### Brief Description of the Drawings

Hereinafter, the embodiments of the invention will be described in detail in connection the accompanying drawings, in which:
Fig. 1 shows the cross-immune effects of GII.4 norovirus virus-like particles (VLP) of the invention on multiple genotypes of noroviruses;
Fig. 2 shows the cross-immune effects of GII.4 norovirus virus-like particles (VLP) of another sequence on multiple genotypes of noroviruses;
Fig. 3 shows the immune intensities of GII.4 norovirus virus-like particles (VLP) of the invention for the genotypes of noroviruses having a cross-immune effect;
Fig. 4 shows the cross-immune effects of various genotypes of virus-like particles comprised in the composition of the invention comprising the virus-like particles of four genotypes of noroviruses GII. 2, GII. 4, GII. 6 and GII. 17 on various genotypes of noroviruses;
Fig. 5 shows the cross-immune effects of the composition of the invention comprising the virus-like particles of four genotypes of noroviruses GII. 2, GII. 4, GII. 6 and GII. 17 on various genotypes of noroviruses;
Fig. 6 shows the immune intensities of the composition of the invention comprising the virus-like particles of four genotypes of noroviruses GII. 2, GII. 4, GII. 6 and GII. 17 on various genotypes of noroviruses having a cross-immune effect.

### Definitions:

Unless defined otherwise, all the scientific and technical terms used herein have the same meaning as understood by one of ordinary skill in the art. With regard to the definitions of terms in the art, a professional can refer specifically to Current Protocols in Molecular Biology (Ausubel). An abbreviation for amino acid residue is a standard 3-letter and/or 1-letter code used in the art to refer to one of the 20 commonly used L-amino acids.

It is further noted that, as used in this description, the term "or" may be used interchangeably with the term "and/or", unless the context clearly indicates otherwise.

As used herein, the terms "pharmaceutical composition", "combination drug", and "drug combination" may be used interchangeably and refer to a combination of at least one drug and optionally a pharmaceutically acceptable excipient or auxiliary material which are combined together to fulfill a certian particular purpose. In certain embodiments, the pharmaceutical composition includes components separated temporally and/or spatially, so long as they are capable of cooperating to achieve an object of the invention. For example, the components contained in the pharmaceutical composition (e.g., GII. 2, GII. 4, GII. 6, GI. 17, or Al(OH)₃) can be administered to a subject as a whole or separately. When the components contained in the pharmaceutical composition are administered separately to a subject, the components may be administered to the subject simultaneously or sequentially.

As used herein, the term "CpG oligodeoxynucleotide" or "CpG-ODN" refers to a short, single-stranded synthetic DNA molecule containing one or more "CpG" units, where C represents cytosine, G represents guanine, and p represents a phosphodiester bond. In particular, the CpG oligodeoxynucleotide is unmethylated. In some embodiments, the CpG-ODN comprises a phosphorothioate linkage or a phosphorothioate backbone. That is, in some embodiments, the CpG-ODN is a phosphorothioate oligodeoxynucleotide (i.e., a thio-oligodeoxynucleotide). Preferably, all the internucleotide linkages in the CpG-ODN are phosphorothioate linkages, i.e., the CpG-ODN is a perthio-oligodeoxynucleotide. In other embodiments, the CpG-ODN comprises two or more copies of 5'-TTCGTT-3' motif or 5'-TCGTCGTCG-3' motif. In particular, the CpG-ODN has a sequence selected from a grpup consisting of TCG TTC GTT CGT TCG TTC GTT (SEQ ID NO: 9), TCG TTC GTT CGT TCG TTC GTT CGT T (SEQ ID NO: 10), or TCG TCG TCG TCG TCG TCG TCG (SEQ ID NO: 11), preferably TCG TTC GTT CGT TCG TTC GTT (SEQ ID NO: 9).

As used herein, a "saponin" refers to an active ingredient found in a corresponding plant, in particular QS21, a quillaja saponin, or Iscom adjuvant, i.e., an immunostimulating complex adjuvant, in particular Iscom matrix (ISCOM MATRIX) that does not comprise an antigen, which is an adjuvant composed of phospholipid, saponin, and cholesterol with a cage-like structure.

As used herein, a "therapeutically effective amount" or an "effective amount" refers to a dosage sufficient to show its benefit to a subject to which it is administered. The actual amount administered, as well as the rate and time course of administration, will depend on the own condition and severity of the subject being treated. A prescription of treatment (e.g., determination of dosage, etc.) is ultimately the responsibility of, and determined by, general practitioners and other physicians, often taking into account the disease being treated, the condition of the individual patient, the site of delivery, the method of administration, and other factors known to physicians.

As used herein, the term "mammal " refers to a human, and may also be other animals, such as wild animals (e.g., herons, storks, cranes, etc.), domestic animals (e.g., ducks, geese, etc.) or laboratory animals (e.g., chimpanzees, monkeys, rats, mice, rabbits, guinea pigs, woodchucks, ground squirrels, etc.).

In other embodiments, the composition of the invention may further comprise an additional additive, such as a pharmaceutically acceptable carrier or additive, particularly when presented as a pharmaceutical formulation.

Preferred pharmaceutical carriers are especially water, buffered aqueous solutions, preferably isotonic saline solutions such as PBS (phosphate buffered solution), glucose, mannitol, dextrose, lactose, starch, magnesium stearate, cellulose, magnesium carbonate, 0.3% glycerol, hyaluronic acid, ethanol or polyalkylene glycols such as polypropylene glycol, triglycerides and the like. The type of pharmaceutical carrier used depends *inter alia* on whether the composition according to the invention is formulated for oral, nasal, intradermal, subcutaneous, intramuscular or intravenous administration. The composition according to the invention may comprise a wetting agent, an emulsifying agent, or a buffer substance as an additive.

The pharmaceutical composition, vaccine or pharmaceutical formulation according to the invention may be administered by any suitable route, e.g. orally, nasally, intradermally, subcutaneously, intramuscularly, or intravenously.

### Specific Embodiments

The invention is further illustrated by the description of the specific embdiments in conjunction with the accompanying drawings, which are not limitation to the invention. A person skilled in the art can make various modifications or improvements in light of the basic concept of the invention, as long as they do not depart from the basic concept of the invention, and such modifications or improvements are within the scope of the invention.

### Example 1 Preparation method of norovirus antigens

Multiple genotypes of norovirus VLP proteins were prepared, respectively, with the amino acid sequences shown below:
GII. 1 norovirus VLP protein of the amino acid sequence shown as SEQ ID NO: 1;
GII. 2 norovirus VLP protein of the amino acid sequence shown as SEQ ID NO: 2;
GII. 3 norovirus VLP protein of the amino acid sequence shown as SEQ ID NO: 3;
GII. 4 norovirus VLP protein of the amino acid sequence shown as SEQ ID NO: 4;
GII. 6 norovirus VLP protein of the amino acid sequence shown as SEQ ID NO: 5;
GII. 7 norovirus VLP protein of the amino acid sequence shown as SEQ ID NO: 6;
GII. 12 norovirus VLP protein of the amino acid sequence shown as SEQ ID NO: 7;
GII. 17 norovirus VLP protein of the amino acid sequence shown as SEQ ID NO: 8.
GII. 4 norovirus VLP protein of the amino acid sequence shown as SEQ ID NO: 12 was used for comparison.

The specific preparation method was as follows: according to the target protein sequence, its nucleic acid sequence was optimized to make its codons suiable for yeast expression system, and the target gene was synthesized. The synthesized target gene was ligated with pMAUR (S.C) KARS1 plasmid by a process of enzyme digestion and ligation, and the same was transformed into Top 10 competent cells. Positive monoclones were picked and sequenced for verification. A large number of monoclonal bacteria cells were amplified, and a large amount of plasmids suiable for electrotransformation were extracted by a plasmid extraction kit. The plasmids were transformed into uracil auxotrophic Hansenula competent cells by a transformation process of electroporation, and the positive transformed cells were obtained by using G418 resistance screening.

The recombinant strains were inoculated into a yeast extract peptone dextrose medium for activation. The activated strains were transferred into a yeast extract peptone dextrose medium for enlarged culture. After the strains were cultured into the plateau phase, methanol was added into the medium for induction culture, once every 24h, twice in total. At the end of induction, the bacteria cells were collected, and the protein was obtained after disrupting the bacteria cells and purification.

### Example 2 Animal immunization experiment

### 2.1 Experimental animals

Balb/c mice, female, 6 weeks old, 18 mice, Shanghai Lingchang Experimental Animal Technology Co. Ltd.

### 2.2 Experimental materials

Various genotypes of norovirus VLP proteins were prepared in Example 1.

Aluminum hydroxide was available from Beijing Baiao Laibo Technology Co. Ltd.

### 2.3 Experiment grouping

**Table 1 Grouping of animal immunization experiment**

| Group | Quantity (mouse) | Component µg/mouse | | |
|---|---|---|---|---|
| | | GII.4 VLP | GII. 4 VLP for comparison | Al(OH)₃ |
| Al(OH)₃ | 6 | | | 100 |
| GII. 4 | 6 | 5 | | 100 |
| GII. 4 for comparison | 6 | | 5 | 100 |

### 2.4 Animal immunity

1) Administration route: intramuscular injection.
2) Administration method: the posterior region of left thigh of the mouse was selected and injected with 100 µl/mouse.
3) Administration frequency and duration: the administration was continued for 3 times, once every 3 weeks, i.e., injection administration at Week 0, Week 3 and Week 6, respectively. Blood was collected two weeks after the first immunization, three weeks after the second immunization, and at the end of two weeks after the third immunization.
4) Serum collection method: the whole blood was placed into a constant temperature incubator at 37°C and let stand for 40-50 min, and let stand at 4°C for 1h, and centrifuged at 12000 rpm at 4°C for 10 min. The supernatant was sucked and cryopreserved in a medical low-temperature freezer for future use.

### Example 3 Cross-blocking experiment of the pharmaceutical composition

### 3.1 Reagent materials

1) BSA (bovine serum albumin, Beyotime), TMB chromogen solution (Thermo), HBGA (Glycotech), rabbit polyvalent antiserum (AbMax), goat anti-rabbit IgG-HRP (horseradish peroxidase labeled goat anti-rabbit IgG, SIGMA), and avidin plate (Thermo);
2) Wash solution: 0.1 M PB buffer;
   Diluent: 0.1 M PB buffer + 0.25% BSA;
   Stopping solution: 2M H₂SO₄;
   They were all formulated by Nanjing Grand Theravac Biopharmaceuticals Co. Ltd.;
3) Norovirus VLP proteins: GI. 1, GII. 2, GII. 3, GII. 4, GII. 6, GII. 7, GII. 12 and GII. 17 were prepared in Example 1, respectively, 8 µg/ml.

### 3.2 Detection steps

1) The avidin plate was washed once with the washing solution at 200 µl/well;
2) HBGA was diluted to 4 µg/ml and added at 100 µl/well to the avidin plate in step 1), followed by incubation at 25°C for 1h;
3) The serum (from Example 2) was diluted from 20-fold to 1280-fold in a 2-fold ratio in a 96-well plate, with a final volume of 60 µl; 60 µl sinle genotype of norovirus VLP protein was added to each well and mixed to form a VLP-serum mixture, and a positive control (no serum) and a blank control (diluent) were set up; incubation was conducted at 4°C for 1h;
4) The avidin plate coated with HBGA in step 2) was washed twice with the washing solution at 200 µl/well; 100 µl of VLP-serum mixture was pipetted from the 96-well plate in step 3) and added into the avidin plate, and incubated at 4°C for 2h;
5) The avidin plate was washed 3 times with the washing solution at 200µl/well, added with the rabbit antiserum (1: 10000), and incubated at 4°C for 1h;
6) The avidin plate was washed 3 times with the washing solution at 200µl/well, added with the goat anti-rabbit IgG-HRP (1: 10000), and incubated at 37°C for 40 min;
7) The avidin plate was washed 3 times with the washing solution at 200 µl/well, and added with the TMB chromogen solution at 100 µl/well; the color development was conducted at 25°C for 5-15 min;
8) Termination: the reaction was stopped by adding the stop solution at 100 µl/well;
9) Reading: OD450nm values were determined (corrected by OD630nm).

Detection standard: blocking index% = (1- A value for Serum group / A value for positive control) x 100%, and BT50, the highest dilution of serum that can block 50% of binding VLP to HBGA, was calculated.

Setting of Cut-off: Two-fold BT50 for Al(OH)₃ group was taken as the critical value to judge whether there was a cross-immune effect.

### 3.3 Experimental results

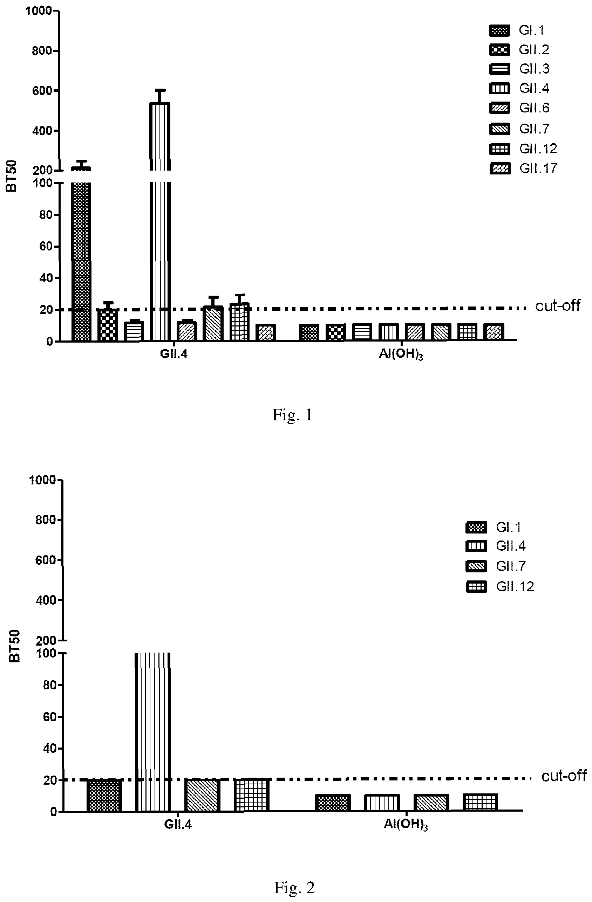

The results are shown in Fig. 1. The GII. 4 norovirus VLP protein provided herein had a significant property of cross immunity against GI. 1, and the BT50 could reach above 250. In addition, it had certain cross-immune effects on GII. 2, GII. 7 and GII. 12. The GII. 4 protein of another sequence as comparison showed no cross-immune effects on GI. 1, GII. 7 and GII. 12 (Fig. 2).

### Example 4 Detection of IgG antibody levels for the pharmaceutical composition

### 4.1 Reagent materials

1) Skimmed milk powder (BD), PBS (ZSGB-Bio), carbonate (SIGMA), Tween 20 (BIO-LINK), TMB chromogen solution (Thermo), horseradish peroxidase-labeled goat anti-rabbit IgG (hereinafter referred to as goat anti-rabbit IgG-HRP, SIGMA);
2) Coating solution: 0.5% (g/100ml) carbonate solution;
   Washing buffer PBST: 10g/L PBS + 0.5 ml/L Tween 20;
   Diluent: 2% skimmed milk PBST;
   Stopping solution: 2M H₂SO₄;

All the above were prepared by Nanjing Grand Theravac Biopharmaceuticals Co. Ltd.

### 4.2 Detection steps

1) Coating: GI. 1, GII. 2, GII. 3, GII. 4, GII. 6, GII. 7, GII. 12 and GII. 17 norovirus VLP proteins were diluted to 1 µg/ml with the coating solution respectively to coat an ELISA enzyme-linked plate at 50 µl/well at 4°C overnight;
2) Washing: the plate was washed with the washing buffer PBST 3 times, 2-3 min/time;
3) Blocking: the blocking solution 5% milk was added at 200 µl/well for blocking at 37°C for 1 h;
4) Washing: the plate was washed by the washing buffer PBST 3 times, 2-3 min/time;
5) First antibody: the double diluted serum (from Example 2) was added, and the dilution was started from 90-fold to 196830-fold in a 3-fold ratio at 50µl/well; two blank control wells were set; the incubation was conducted at 37°C for 1h;
6) Washing: the plate was washed with the washing buffer PBST 3 times, 2-3 min/time;
7) Second antibody: HRP-goat anti-mouse IgG antibody (1: 10000) was incubated at 37°C for 40 min;
8) Washing: the plate was washed with the washing buffer PBST 3 times, 2-3 min/time;
9) Color development: the TMB chromogen solution was added at 50µl/well; the color development was conducted for 10 min;
10) Termination: the reaction was stopped with the stop solution at 50 µl/well;
11) Readings: the OD450nm values were determined (corrected by OD630nm).

### 3.6. Experimental results

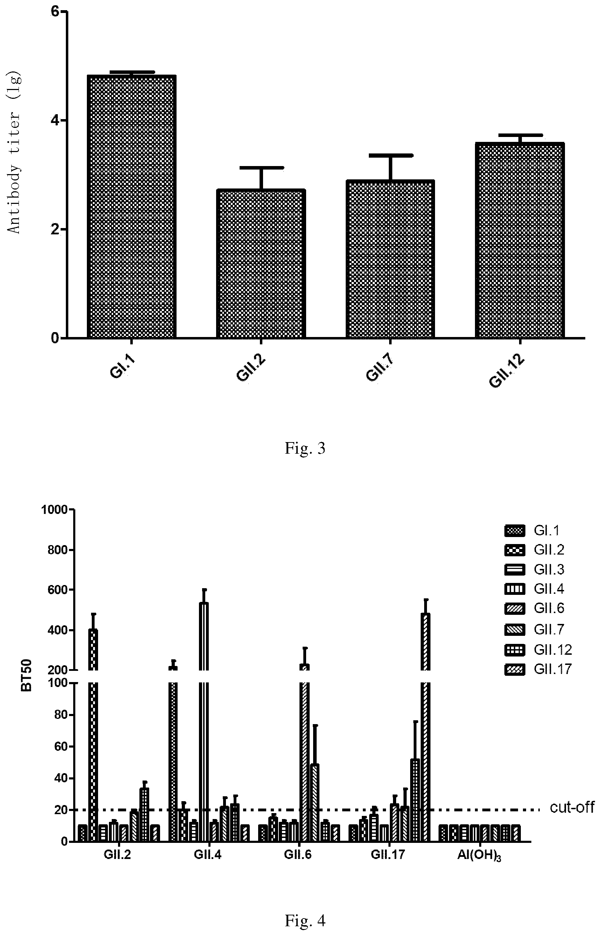

The results are shown in Fig. 3. The GII. 4 virus-like particle VLP provided herein had immune responses to GI. 1, GII. 2, GII. 7 and GII. 12, wherein the antibody titer against GI. 1 could reach a level of above 10,000.

In summary, provided is use of the virus-like particle VLP of GII. 4 norovirus in the preparation of a composition and a kit for the prevention, treatment and/or diagnosis of infection by any one or more of GI. 1, GII. 2, GII. 7 and GII. 12 noroviruses. It not only achieves the cross-immune effect between GII and GI genomes, but also has cross-immune effects on GII. 2, GII. 7 and GII. 12, and can achieve a goal of covering multiple genotypes with a single antigen, reduce the process difficulty, decrease the production cost, and has a broad market prospect.

### Example 5 Animal immunization experiment

### 2.1 Experimental animals

Balb/c mice, female, 6 weeks old, 36 mice, Shanghai Lingchang Experimental Animal Technology Co. Ltd.

### 2.2 Experimental materials

Multiple genotypes of norovirus VLP proteins were prepared in Example 1.

Aluminum hydroxide was available from Beijing Baiao Laibo Technology Co. Ltd.

### 2.3 Experiment grouping

**Table 1 Grouping of animal immunization experiment**

| Group | Quantity (mouse) | Component µg/mouse | | | | |
|---|---|---|---|---|---|---|
| | | GII. 2 VLP | GII. 4 VLP | GII. 6 VLP | GII. 17 VLP | Al(OH)₃ |
| Al(OH)₃ | 6 | | | | | 100 |
| GII.2 | 6 | 5 | | | | 100 |
| GII.4 | 6 | | 5 | | | 100 |
| GII.6 | 6 | | | 5 | | 100 |
| GII.17 | 6 | | | | 5 | 100 |
| Composition | 6 | 5 | 5 | 5 | 5 | 100 |

### 2.4 Animal immunization

1) Administration route : intramuscular injection.
2) Administration method: the posterior region of left thigh of the mouse was selected and injected with 100 µl/mouse.
3) Administration frequency and duration: the administration continued for 3 times, once every 3 weeks, i.e., injection administration at Week 0, Week 3 and Week 6, respectively. Blood was collected two weeks after the first immunization, three weeks after the second immunization, and at the end of two weeks after the third immunization.
4) Serum collection method: the whole blood was placed into a constant temperature incubator at 37°C and let stand for 40-50 min, let stand at 4°C for 1h, and centrifuged at 12000 rpm at 4°C for 10 min. The supernatant was sucked and cryopreserved in a medical low-temperature freezer for future use.

### Example 6 Cross-blocking experiment for the pharmaceutical composition

Reagent materials and detection steps were the same as those described in Example 3. The serum samples used in the experiment were from Example 5.

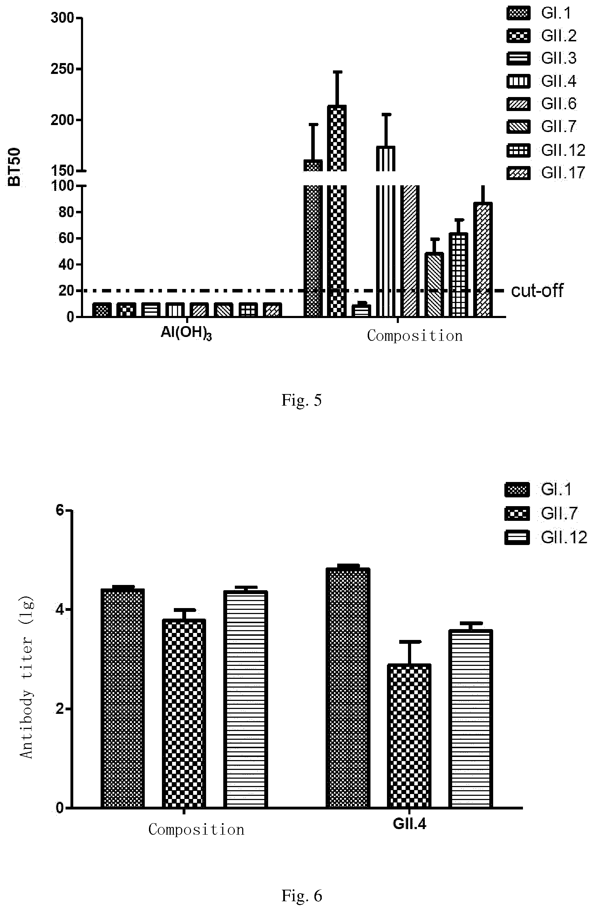

The experimental results are shown in Figs. 4 and 5. Fig. 4 shows the cross-immune effect of each individual antigen. It can be seen that GII. 2 had a cross-immune effect on GII. 12, GII. 4 had cross-immune effects on GI. 1, GII. 2, GII. 7 and GII. 12, GII. 6 had a cross-immune effect on GII. 7, and GII. 17 had cross-immune effects on GII. 6, GII. 7 and GII. 12.

Fig. 5 shows that the composition comprising the four genotypes of noroviruses VLP proteins GII. 2, GII. 4, GII. 6 and GII. 17 provided herein could cover seven noroviruses, wherein GI. 1, GII. 7 and GII. 12 increased the covered types due to cross immune effects. The cross immune effect on GI. 1 was the strongest and the BT50 could reach above 150.

### Example 7 Detection of IgG antibody levels of the pharmaceutical composition

Reagent materials and detection steps were the same as those described in Example 4. The serum samples used in the experiment were from Example 5.

The experimental results are shown in Fig 6. The pharmaceutical composition comprising virus-like particles of the four genotypes of noroviruses GII. 2, GII. 4, GII. 6 and GII. 17 had immune responses to GI. 1, GII. 7 and GII. 12, and the antibody titers of above 10000 could be achieved. Compared with the IgG antibody levels of a single GII. 4 antigen for various genotypes, those for both GII. 7 and GII. 12 were increased by more than 10 folds, achieving the synergy of antigen cross-interaction.

In summary, the composition comprising virus-like particles of the four genotypes of noroviruses GII. 2, GII. 4, GII. 6 and GII. 17 of the invention can not only achieve a goal of preventing infections by seven genotypes of noroviruses by the cross-immune effects of the four antigens, but also have synergy of immune effects for the same genotypes to which the four antigens target, so as to enhance the antibody levels against the genotyes covered, reduce the types of antigens, and decrease the process difficulty. It has a very high clinical application value and broad market prospect.

Although the invention has been described in detail above, those skilled in the art will appreciate that various modifications and variations can be made to the invention without departing from the spirit and scope of the invention. The claimed scope of the invention is not limited by the foregoing detailed description, and such modifications and variations are intended to fall within the scope of the claims. While only the examples of specific embodiments of the invention have been described above, it will be appreciated by those skilled in the art that the foregoing is illustrative only and that the scope of the invention is defined by the appended claims. Those skilled in the art may make a great variety of changes or modifications to these embodiments without departing from the principles and substance of the invention, but these changes or modifications shall fall within the protection scope of the invention.

## Claims

1. A G11.4 norovirus virus-like particle or active fragment thereof for inducing an immune response to multiple genotypes of noroviruses in an animal, wherein the G11.4 norovirus virus-like particle or active fragment thereof comprises or is composed of one amino acid sequence selected from a group consisting of:
(1) an amino acid sequence shown as SEQ ID NO: 4;
(2) an amino acid sequence having an identity of at least 90%, 91%, 92%, 93%, 94%, 95%, 96%, 97%, 98%, 99% or more to the amino acid sequence shown as SEQ ID NO: 4;
(3) an amino acid sequence having an amino acid substitution, deletion or insertion at one or more sites in the amino acid sequence shown as SEQ ID NO: 4; and
(4) a segment of consecutive amino acids from position X to position 480 in the amino acid sequence shown as SEQ ID NO: 4, wherein X is an integer between 2 and 39; preferably, X is 27, 37 or 39.

2. The norovirus virus-like particle or active fragment thereof according to claim 1, wherein the animal is a mammal, preferably a human; and/or
the multiple genotypes of noroviruses include two or more selected from a group consisting of GI. 1, GII. 2, GII. 4, GII. 7 and GII. 12 noroviruses.

3. A composition for inducing an immune response to a norovirus in an animal, comprising the G11.4 norovirus virus-like particle or active fragment thereof according to claim 1.

4. The composition according to claim 3, wherein the animal is a mammal, preferably a human.

5. The composition according to claim 3 or 4, wherein the norovirus is one or more selected from a group consistingof GI. 1, GII. 2, GII. 4, GII. 7 and GII. 12 noroviruses.

6. A composition for inducing an immune response to a norovirus, comprising GII. 2, GII. 4, GII. 6 and GII. 17 noroviruses virus-like particles or active fragments thereof.

7. The composition according to claim 6, wherein the GII. 2 norovirus virus-like particle or active fragment thereof comprises or is composed of one amino acid sequence selected from a group consisting of:
(1) an amino acid sequence shown as SEQ ID NO: 2;
(2) an amino acid sequence having an identity of at least 90%, 91%, 92%, 93%, 94%, 95%, 96%, 97%, 98%, 99% or more to the amino acid sequence shown as SEQ ID NO: 2; and
(3) an amino acid sequence having an amino acid substitution, deletion or insertion at one or more sites in the amino acid sequence shown as SEQ ID NO: 2;
preferably, the G11.4 norovirus virus-like particle or active fragment thereof comprises or is composed of one amino acid sequence selected from a group consisting of:
(1) an amino acid sequence shown as SEQ ID NO: 4;
(2) an amino acid sequence having an identity of at least 90%, 91%, 92%, 93%, 94%, 95%, 96%, 97%, 98%, 99% or more to the amino acid sequence shown as SEQ ID NO: 4;
(3) an amino acid sequence having an amino acid substitution, deletion or insertion at one or more sites in the amino acid sequence shown as SEQ ID NO: 4; and
(4) a segment of consecutive amino acids from position X to position 480 of the amino acid sequence shown as SEQ ID NO: 4, wherein X is an integer between 2 and 39; preferably, X is 27, 37 or 39;
preferably, the GII. 6 norovirus virus-like particle or active fragment thereof comprises or is composed of one amino acid sequence selected from a group consisting of:
(1) an amino acid sequence shown as SEQ ID NO: 5;
(2) an amino acid sequence having an identity of at least 90%, 91%, 92%, 93%, 94%, 95%, 96%, 97%, 98%, 99% or more to the amino acid sequence shown as SEQ ID NO: 5;
(3) an amino acid sequence having an amino acid substitution, deletion or insertion at one or more sites in the amino acid sequence shown as SEQ ID NO: 5; and
(4) a segment of consecutive amino acids from position X to position 547 of the amino acid sequence shown as SEQ ID NO: 5, wherein X is an integer between 2 and 27; preferably, X is 27;
preferably, the GII. 17 norovirus virus-like particle or active fragment thereof comprises or is composed of one amino acid sequence selected from a group consisting of:
(1) an amino acid sequence shown as SEQ ID NO: 8;
(2) an amino acid sequence having an identity of at least 90%, 91%, 92%, 93%, 94%, 95%, 96%, 97%, 98%, 99% or more to the amino acid sequence shown as SEQ ID NO: 8; and
(3) an amino acid sequence having an amino acid substitution, deletion or insertion at one or more sites in the amino acid sequence shown as SEQ ID NO: 8.

8. The composition according to claim 6 or 7, wherein the GII. 2 norovirus virus-like particle or active fragment thereof comprises or is composed of an amino acid sequence as shown as SEQ ID NO: 2; the G11.4 norovirus virus-like particle or active fragment thereof comprises or is composed of an amino acid sequence as shown as SEQ ID NO: 4; the GII. 6 norovirus virus-like particle or active fragment thereof comprises or is composed of an amino acid sequence as shown as SEQ ID NO: 5; and the GII. 17 norovirus virus-like particle or active fragment thereof comprises or is composed of an amino acid sequence as shown as SEQ ID NO: 8.

9. The composition according to any one of claims 6 to 8, wherein the norovirus is one or more selected from a group consisting of GI. 1, GII. 2, GII. 4, GII. 6, GII. 7, GII. 12 and GII. 17 noroviruses.

10. The composition according to any one of claims 3 to 9, wherein the composition is a pharmaceutical composition, such as a vaccine, for the prevention and/or treatment of a norovirus infection.

11. The composition according to claim 10, wherein the pharmaceutical composition further comprises one or more adjuvant selected from a group consisting of an aluminum adjuvant, a TRL adjuvant, and a saponin adjuvant;
preferably, the aluminum adjuvant is one or more selected from a group consisting of aluminum hydroxide, aluminum phosphate and aluminum sulphate; more preferably, the aluminum adjuvant is aluminum hydroxide;
more preferably, the TRL adjuvant is TRL9 adjuvant; more preferably, the TRL9 adjuvant is a CPG adjuvant; further more preferably, the nucleotide sequence of the CPG adjuvant is one selected from a group consisting of SEQ ID NO: 9, SEQ ID NO: 10 and SEQ ID NO: 11; preferably, the saponin adjuvant is QS21 or Iscom adjuvant.

12. The composition according to claim 10 or 11, wherein the norovirus infection is gastroenteritis, preferably viral acute gastroenteritis.

13. An immue kit comprising the norovirus virus-like particle or active fragment thereof according to claim 1 or 2, or the composition according to any one of claims 3 to 9;
preferably, the kit is a kit for detecting a norovirus.

14. Use of the norovirus virus-like particle or active fragment thereof according to claim 1 or 2, the composition according to any one of claims 3 to 12, or the immune kit according to claim 13, in the preparation of a product as follows:
(1) a medicament for the prophylaxis and/or treatment of a norovirus infection;
(2) a kit for diagnosing a norovirus infection; or
(3) an immunogen for the development of a norovirus antibody.

15. The use according to claim 14, wherein the norovirus infection is gastroenteritis, preferably viral acute gastroenteritis.
